# DEMANDE DE BREVET EUROPEEN

(11) **EP 2 338 467 A1**
(43) Date de publication de la demande: **29.06.2011**
(21) Numéro de dépôt: 10196188.6
(22) Date de dépôt: 21.12.2010
(51) Int. Cl.: A61K 8/31, A61K 8/34, A61K 8/895, A61Q 5/12

(54) **Composition cosmetique comprenant au moins un alcane lineaire volatil, au moins une silicone et au moins un corps gras dans un rapport corps gras/silicone particulier**

(30) Priorité: 23.12.2009 FR 0959556
(71) Demandeur: L'Oréal, 75008 Paris (FR)
(72) Inventeur: Desenne, Patricia, 74370, Pringy (FR); Chesneau, Laurent, 92300, Levallois (FR)
(74) Mandataire: Dossmann, Gérard

(57) **Abrégé**

La présente invention concerne une composition cosmétique comprenant dans un milieu aqueux :
- de 0,1 à 20% en poids d'un ou plusieurs alcanes linéaires volatils,
- de 0,5 à 25% en poids d'une ou plusieurs silicones de viscosité supérieure ou égale à 20 000 mm²/s,
- un ou plusieurs corps gras non siliconés,

en un rapport pondéral corps gras non siliconé(s)/silicone(s) de viscosité supérieure ou égale à 20 000 mm²/s inférieur ou égal à 5.

Elle concerne aussi son utilisation pour le traitement cosmétique des matières kératiniques, de préférence des fibres kératiniques comme les cheveux, et un procédé de traitement cosmétique des matières kératiniques mettant en oeuvre ladite composition.

## Description

La présente invention concerne une composition cosmétique comprenant dans un milieu aqueux un ou plusieurs alcanes linéaires volatils, une ou plusieurs silicones de viscosité élevée, et un ou plusieurs corps gras non siliconés dans des proportions particulières, son utilisation pour le traitement cosmétique des matières kératiniques, de préférence des fibres kératiniques comme les cheveux et un procédé de traitement cosmétique des matières kératiniques mettant en oeuvre ladite composition.

Dans le domaine du traitement capillaire, l'utilisation de solvants volatils est connue dans des produits capillaires de soins rincés ou non rincés. Ils sont généralement utilisés pour différentes raisons. Ils permettent notamment de modifier le rendu sensoriel d'un produit capillaire en lui conférant une texture légère et non collante dans la main. Ils peuvent également lui conférer un caractère glissant qui facilite la répartition du produit sur les cheveux et en particulier sur des cheveux secs.

Dans les émulsions aqueuses de type huile-dans-eau, qui peuvent se présenter sous forme de crèmes plus ou moins gélifiées, l'ajout de solvants volatils peut également permettre de solubiliser des gommes de silicone qui, de par leur viscosité intrinsèque, seraient difficiles à introduire dans les compositions.

Ces solvants volatils qui sont généralement des esters gras liquides, des huiles hydrocarbonées de type isododécane ou isohexadécane, et/ou des huiles siliconées, peuvent notamment induire des problèmes de toucher gras, de manque de brillance, de cheveux raidis, durs.

Il subsiste donc un besoin de remplacer ces solvants volatils pour éviter les inconvénients cités ci-dessus.

La demanderesse a maintenant découvert de façon inattendue et surprenante, que l'association d'un ou plusieurs alcanes linéaires volatils, d'une ou plusieurs silicone de viscosité élevée, et d'un ou plusieurs corps gras non siliconés dans des proportions particulières dans une composition aqueuse permettait d'éviter les inconvénients mentionnés ci-dessus et d'améliorer les propriétés cosmétiques telles que le lissage, la souplesse, le démêlage, le volume (notamment le décollement de racines), et la tonicité de la chevelure.

En particulier, la composition selon l'invention permet d'obtenir des cheveux plus lisses, homogènes et/ou plus souples, au moment du rinçage. Sur cheveux humides, on obtient des cheveux plus faciles à démêler ou plus toniques et/ou ayant des racines plus décollées (en racines, les cheveux ne sont pas plaqués sur le cuir chevelu mais forment un angle, d'où un apport de volume). En outre, sur cheveux secs, on obtient des cheveux plus souples et/ou plus lisses au toucher.

Ainsi, l'invention a pour objet une composition cosmétique comprenant, dans un milieu aqueux :
- de 0,1 à 20% en poids d'un ou plusieurs alcanes linéaires volatils,
- de 0,5 à 25% en poids d'un ou plusieurs silicones de viscosité supérieure ou égale à 20 000 mm²/s à 25°C,
- un ou plusieurs corps gras non siliconés différents des alcanes linéaires volatils,

le rapport pondéral de la quantité dudit ou desdits corps gras non siliconés différents des alcanes linéaires volatils, sur la quantité de ladite ou desdites silicones étant inférieur ou égal à 5.

Elle a encore pour objet l'utilisation de ladite composition pour le traitement cosmétique des matières kératiniques, de préférence des fibres kératiniques comme les cheveux, notamment comme produit capillaire de soin rincé.

Un autre objet de l'invention est un procédé de traitement cosmétique des matières kératiniques, de préférence des fibres kératiniques comme les cheveux, mettant en oeuvre ladite composition.

La composition cosmétique selon l'invention comprend, dans un milieu aqueux :
- de 0,1 à 20% en poids d'un ou plusieurs alcanes linéaires volatils,
- de 0,5 à 25% en poids d'une ou plusieurs silicones de viscosité supérieure ou égale à 20 000 mm²/s,
- un ou plusieurs corps gras non siliconés différents des alcanes linéaires volatils,
le rapport pondéral de la quantité dudit ou desdits corps gras non siliconés différents des alcanes linéaires volatils, sur la quantité de ladite ou desdites silicones étant inférieur ou égal à 5, et de préférence inférieur ou égal à 3.

Ledit rapport va de préférence de 0,01 à 5, plus préférentiellement de 0,1 à 5, et mieux encore de 0,1 à 3.

Par « un ou plusieurs alcane(s) linéaire(s) volatil(s) », on entend indifféremment « une ou plusieurs huile(s) alcane(s) linéaire(s) volatile(s) ».

Un alcane linéaire volatil convenant à l'invention est liquide à température ambiante (environ 25 °C) et sous pression atmosphérique (101 325 Pa ou 760 mm Hg).

Par « alcane linéaire volatil » convenant à l'invention, on entend un alcane linéaire, susceptible de s'évaporer au contact de la peau en moins d'une heure, à température ambiante (25°C) et sous pression atmosphérique (101 325 Pa), liquide à température ambiante, ayant notamment une vitesse d'évaporation allant de 0,01 à 15 mg/cm²/min, à température ambiante (25°C) et sous pression atmosphérique (101 325 Pa).

De préférence, le ou les alcanes linéaires volatils convenant à l'invention présentent une vitesse d'évaporation allant de 0,01 à 3,5 mg/cm²/min, mieux de 0,01 à 1,5 mg/cm²/min, à température ambiante (25°C) et sous pression atmosphérique (101 325 Pa).

De façon plus préférée, le ou les alcanes linéaires volatils convenant à l'invention présentent une vitesse d'évaporation allant de 0,01 à 0,8 mg/cm²/min, préférentiellement de 0,01 à 0,3 mg/cm²/min, et encore plus préférentiellement de 0,01 à 0,12 mg/cm²/min, à température ambiante (25°C) et sous pression atmosphérique (101 325 Pa).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m³ régulée en température (25 °C) et en hygrométrie (humidité relative 50 %), 15 g de solvant hydrocarboné volatil.

On laisse le solvant hydrocarboné volatil s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm²) en fonction du temps (en min).

Puis on calcule la vitesse d'évaporation qui correspond à la tangente à l'origine de la courbe obtenue. Les vitesses d'évaporation sont exprimées en mg de solvant volatil évaporé par unité de surface (cm²) et par unité de temps (minute).

Selon un mode de réalisation préféré, le ou les alcanes linéaires volatils convenant à l'invention ont une pression de vapeur (appelée également pression de vapeur saturante) non nulle, à température ambiante, en particulier une pression de vapeur allant de 0,3 Pa à 6000 Pa.

De façon préférée, le ou les alcanes linéaires volatils convenant à l'invention ont une pression de vapeur allant de 0,3 à 2000 Pa, mieux de 0,3 à 1000 Pa, à température ambiante (25°C).

De façon plus préférée, le ou les alcanes linéaires volatils convenant à l'invention ont une pression de vapeur allant de 0,4 à 600 Pa, préférentiellement de 1 à 200 Pa, et encore plus préférentiellement de 3 à 60 Pa, à température ambiante (25°C).

Selon un mode de réalisation, un alcane linéaire volatil convenant à l'invention peut présenter un point éclair compris dans l'intervalle variant de 30 à 120 °C, et plus particulièrement de 40 à 100 °C. Le point éclair est en particulier mesuré selon la Norme iso 3679.

Selon un mode de réalisation, le ou les alcanes linéaires volatils convenant dans l'invention peuvent être les alcanes linéaires comportant de 7 à 15 atomes de carbone, de préférence de 8 à 14 atomes de carbone, et mieux de 9 à 14 atomes de carbone.

De façon plus préférée, le ou les alcanes linéaires volatils convenant dans l'invention comprennent de 10 à 14 atomes de carbone, et encore plus préférentiellement de 11 à 14 atomes de carbone.

Le ou les alcanes linéaires volatils convenant à l'invention peuvent être avantageusement d'origine végétale.

De préférence, l'alcane linéaire volatil ou le mélange d'alcanes linéaires volatils présent dans la composition selon l'invention comprend au moins un isotope ¹⁴ C du carbone (carbone 14). En particulier, l'isotope ¹⁴C peut être présent en un ratio isotopique ¹⁴C/¹²C (en nombre d'isotopes) supérieur ou égal à 1.10⁻¹⁶, de préférence supérieur ou égal à 1.10⁻¹⁵ , de préférence encore supérieur ou égal 7,5.10⁻¹⁴, et mieux supérieur ou égal 1,5.10⁻¹³. De préférence, le ratio isotopique 14C / ¹²C va de 6 10⁻¹³ , à 1,2.10⁻¹².

La quantité d'isotopes ¹⁴C dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane ou mélange d'alcanes peut être obtenu, directement ou en plusieurs étapes, à partir d'une matière première végétale comme une huile, un beurre, une cire, etc.

A titre d'exemple d'alcanes convenant à l'invention, on peut mentionner les alcanes décrits dans les demandes de brevets WO 2007/068371 et W02008/155059. Ces alcanes sont obtenus à partir d'alcools gras, eux-mêmes obtenus à partir d'huile de coprah ou de palme.

A titre d'exemples d'alcanes linéaires convenant à l'invention, on peut citer le n-heptane (C7), le n-octane (C8), le n-nonane (C9), le n-décane (C10), le n-undécane (C11), le n-dodécane (C12), le n-tridécane (C13), le n-tétradecane (C14), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode de réalisation préféré, on peut citer les mélanges de n-undécane (C11) et de n-tridécane (C13) obtenus notamment aux exemples 1 et 2 de la demande W02008/155059.

On peut également citer le n-dodécane (C12) et le n-tétradécane (C 14) vendus respectivement sous les références PARAFOL 12-97 et PARAFOL 14-97 par la société Sasol, ainsi que leurs mélanges.

Un mode de réalisation consiste à utiliser un seul alcane linéaire volatil.

Alternativement, on peut utiliser un mélange d'au moins deux alcanes linéaires volatils distincts, différant entre eux d'un nombre de carbone n d'au moins 1, en particulier différant entre eux d'un nombre de carbone de 1 ou de 2.

Selon un mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils C10/C11, C11/C12, ou C12/C13.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils C10/C12, ou C12/C14, pour un nombre de carbone n pair et le mélange C11/C13 pour un nombre de carbone n impair.

Selon un mode de réalisation préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils C11 /C13 ou un mélange d'alcanes linéaires volatils C12/C14.

D'autres mélanges associant plus de 2 alcanes linéaires volatils selon l'invention, tels que, par exemple, un mélange d'au moins 3 alcanes linéaires volatils comportant de 7 à 15 atomes de carbone distincts et différant entre eux d'un nombre de carbone d'au moins 1, peuvent être utilisés dans l'invention.

Dans le cas des mélanges de deux alcanes linéaires volatils, lesdits deux alcanes linéaires volatils représentent de préférence plus de 95%, et mieux plus de 99% en poids du mélange.

Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :
- de 50 à 90% en poids, de préférence de 55 à 80% en poids, préférentiellement encore de 60 à 75% en poids d'alcane linéaire volatil en Cₙ avec n allant de 7 à 15
- de 10 à 50% en poids, de préférence de 20 à 45% en poids, de préférence de 24 à 40% en poids, d'alcane linéaire volatil en
   Cₙ₊ₓ avec x supérieur ou égal à 1, de préférence x=1 ou x=2, avec n+x compris entre 8 et 14,
par rapport au poids total des alcanes dans ledit mélange.

En particulier, ledit mélange d'alcanes linéaires volatils peut contenir en outre :
- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1 % en poids et préférentiellement moins de 0,1% en poids d'hydrocarbures insaturés,

lesdits pourcentages étant exprimés par rapport au poids total du mélange.

Plus particulièrement, les alcanes linéaires volatils convenant dans l'invention peuvent être mis en oeuvre sous la forme d'un mélange n-undécane/n-tridécane.

En particulier, on utilisera un mélange d'alcanes linéaires volatils comprenant :
- de 55 à 80% en poids, de préférence de 60 à 75% en poids d'alcane linéaire volatil en C11 (n-undécane) et
- de 20 à 45% en poids, de préférence de 24 à 40% en poids d'alcane linéaire volatil en C13 (n-tridécane),
par rapport au poids total des alcanes dans ledit mélange.

Selon un mode de réalisation particulier, le mélange d'alcanes est un mélange n-undécane/n-tridécane. En particulier, un tel mélange peut être obtenu selon l'exemple 1 ou l'exemple 2 de la demande WO 2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane, de préférence dans un rapport 85/15 tel que le mélange commercialisé sous la dénomination VEGELIGHT 1214 par la société Biosynthis.

La composition de l'invention comprend de préférence de 1% à 20% en poids d'alcane(s) linéaire(s) volatil(s), en particulier de 1% à 15% en poids, et plus particulièrement de 3% à 15% en poids d'alcane(s) linéaire(s) volatil(s), par rapport au poids total de la composition.

Par simplification, on désigne par « viscosité » la viscosité cinématique du composé.

La ou les silicones utilisables conformément à la présente invention présentent une viscosité supérieure ou égale à 20 000 mm²/s, de préférence supérieure ou égale à 50 000 mm²/s, et mieux encore supérieure ou égale à 100 000 mm²/s. En général, elles présentent une viscosité inférieure à 200 000 000 mm²/s.

De manière plus préférée, on choisit la ou les silicones parmi les silicones présentant une viscosité allant de 100 000 à 150 000 000 mm²/s, de préférence allant de 1 000 000 à 50 000 000 mm²/s.

La viscosité de la silicone est mesurée de préférence à l'aide de rhéomètre de Poiseuille, à une température de 25°C, selon la norme ASTM-D445-97. On peut aussi utiliser la méthode dite de la chute de bille. Lorsque la silicone est utilisée en mélange dans un solvant ou sous la forme d'une émulsion, on mesure la viscosité de la silicone seule, indépendamment du solvant de mélange.

La ou les silicones utilisables dans la composition selon l'invention sont en particulier des polyorganosiloxanes qui peuvent se présenter sous forme de solutions aqueuses, c'est-à-dire solubilisées, ou éventuellement sous forme de dispersions ou micro-dispersions, ou d'émulsions aqueuses. Les polyorganosiloxanes peuvent également se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Dans le cadre de la présente invention, on entend par silicone de viscosité supérieure ou égale à 20 000 mm²/s :
(i) les polyalkylsiloxanes ; parmi les polyalkylsiloxanes, on peut citer principalement les polydiméthylsiloxanes linéaires à groupements terminaux triméthylsilyle, et les polydiméthylsiloxanes à groupements terminaux hydroxydiméthysilyle par exemple;
(ii) les polyarylsiloxanes ;
(iii) les polyalkylarylsiloxanes ; on peut citer les polyméthylphénylsiloxanes, les polydiméthylméthylphénylsiloxanes, les polydiméthyldiphénylsiloxanes linéaires et ramifiés,
   Et en particulier,
(iv) les gommes de silicone tels que les gommes des silicones (i), (ii) et (iii); ce sont des polydiorganosiloxanes de masse moléculaire comprise entre 200 000 et 5 000 000, utilisés seuls ou en mélange dans un solvant choisi parmi les silicones volatiles, les huiles polydiméthylsiloxanes (PDMS), les huiles polyphénylméthylsiloxanes, (PPMS), les isoparaffines, le chlorure de méthylène, le pentane ou leurs mélanges; elles peuvent par exemple, posséder l'une des structures :
   - polydiméthylsiloxane,
   - poly[(diméthylsiloxane)/(méthylvinylsiloxane)],
   - poly[(diméthylsiloxane)/(vinylhydrogénosiloxane)],
   - poly[(dihydrogénodiméthylsiloxane)/(divinylsiloxane)],
   - poly[(diméthylsiloxane)/(diphénylsiloxane)],
   - poly[(diméthylsiloxane))(phénylméthylsiloxane)],
   - poly[(diméthylsiloxane)/(diphénylsiloxane)/(méthylvinyl-siloxane)];
   on peut aussi citer, par exemple, à titre non limitatif, les mélanges suivants :
   1) les mélanges formés à partir d'un polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit "Q2 1401" vendu par la Société DOW CORNING;
   2) les mélanges formés à partir d'une gomme polydiméthylsiloxane avec une silicone cyclique, tel que le produit "SF 1214 SILICONE FLUID" de GENERAL ELECTRIC, qui est une gomme SE 30 de PM 500 000 solubilisée dans la "SF 1202 SILICONE FLUID" (décaméthylcyclopentasiloxane);
   3) les mélanges de deux PDMS de viscosité différente, notamment d'une gomme PDMS et d'une huile PDMS, tels que les produits "SF 1236" et "CF 1241" de la Société GENERAL ELECTRIC;
(v) les résines de silicone ; de préférence des systèmes siloxaniques réticulés renfermant les unités R2Si02/2, RSi03/2 et Si04/2 dans lesquelles R représente un groupement hydrocarboné possédant 1 à 6 atomes de carbone ou un groupement phényle. Parmi ces résines, on peut citer le produit vendu sous la dénomination "DOW CORNING 593";
(vi) ou leurs mélanges.

Ces silicones peuvent être utilisées telles quelles ou sous forme de solutions dans des solvants organiques ou encore sous forme d'émulsions ou de microémulsions.

De préférence, la silicone se trouve sous la forme d'émulsion aqueuse.

Par « émulsion aqueuse », on entend une émulsion de type huile-dans-eau dans laquelle le copolymère siliconé est dispersé sous forme de particules ou de gouttelettes dans la phase aqueuse formant la phase continue de l'émulsion.

Cette émulsion peut être stabilisée par un système émulsionnant usuel.

Cette émulsion de silicone peut avoir une taille de gouttelettes ou de particules de silicone allant de 10 nm à 50 µm, et de préférence de 0,3 µm à 20 µm.

La taille des particules est mesurée par granulométrie laser.

Le système émulsionnant comprend des tensioactifs employés habituellement dans les émulsions de silicone. Ces tensioactifs peuvent être des tensioactifs non-ioniques, cationiques, anioniques ou amphotères ou leurs mélanges.

Le système émulsionnant représente de préférence de 0,5% à 10% en poids par rapport au poids total de l'émulsion.

Le ou les polyorganosiloxanes préférés utilisables selon l'invention sont notamment les produits appartenant à la classe (i) et particulièrement à la sous-classe (iv).

Dans un mode de réalisation préféré, la ou les silicones utilisables selon l'invention sont choisies parmi les gommes de silicones telles que décrites précédemment, et plus préférentiellement celles possédant une structure poly[(dihydrogénodiméthylsiloxane)/(divinylsiloxane)].

De telles émulsions sont notamment commercialisées sous la dénomination DC2-1997 cationic emulsion par la société DOW CORNING. Cette émulsion comprend un copolymère alpha, oméga-vinyl diméthicone/ alpha, oméga-hydrogénodiméthicone ayant une viscosité cinématique d'environ 20.10⁶ cSt (centistokes), un émulsionnant de type cationique tel que le chlorure de cétyltriméthylammonium, un stabilisant de type hydroxyéthylcellulose et de l'eau.

La ou les silicones de viscosité supérieure ou égale à 20 000 mm²/s sont présentes de préférence dans des proportions allant de 0,1% à 10% en poids par rapport au poids total de la composition, et préférentiellement de 0,5% à 5% en poids par rapport au poids total de la composition.

Comme expliqué précédemment, la composition selon l'invention comprend également un ou plusieurs corps gras non siliconés. Les corps gras non siliconés utilisés selon l'invention sont différents des alcanes linéaires volatils précédemment définis.

Par « corps gras », on entend, un composé organique insoluble dans l'eau à température ordinaire (25°C) et à pression atmosphérique (760 mm de Hg), c'est-à-dire de solubilité inférieure à 5% et de préférence à 1% encore plus préférentiellement à 0,1%. Les corps gras non siliconés présentent dans leur structure une chaîne hydrocarbonée comportant au moins 6 atomes de carbone et ne comprenant pas de groupe siloxane. En outre, les corps gras sont généralement solubles dans des solvants organiques dans les mêmes conditions de température et de pression, comme par exemple le chloroforme, l'éthanol, le benzène, l'huile de vaseline ou le décaméthylcyclopentasiloxane.

De préférence, le ou les corps gras non siliconés sont choisis parmi les alcools gras en C₈-C₄₀, les esters d'acide gras en C₈-C₄₀ et/ou d'alcool gras en C₈-C₄₀, les cires, les huiles végétales, animales, minérales et synthétiques, différentes des alcanes linéaires volatils tels que définis précédemment.

Le ou les alcools gras enC₈-C₄₀ peuvent être choisis parmi les alcools de formule R'OH, où R' désigne un radical saturé ou insaturé, linéaire ou ramifié, comportant de 8 à 40 atomes de carbone, de préférence 8 à 30 atomes de carbone. R' désigne de préférence un groupement alkyle en C₁₂-C₂₄ ou alcényle en C₁₂-C₂₄. R' peut être substitué par un ou plusieurs groupements hydroxy.

Le ou les alcools gras peuvent être en particulier choisis parmi l'alcool laurique, l'hexyldécanol, le 2-octyldodécanol, l'alcool cétylique, l'alcool dodécylique, l'alcool décylique, l'alcool stéarylique, l'alcool oléïque, l'alcool béhénique, l'alcool linoléique, l'alcool undécylénique, l'alcool palmitoléïque, l'alcool arachidonique, l'alcool myristylique et l'alcool érucique. On peut également utiliser un mélange d'alcools gras, ce qui signifie que dans un produit commercial peuvent coexister plusieurs espèces d'alcools gras, sous forme d'un mélange. A titre de mélange d'alcools gras, on peut citer l'alcool cétylstéarylique ou cétéarylique.

De manière plus préférée, le ou les alcools gras sont choisis parmi les alcools laurique, myristique, cétylique, stéarique, cétylstéarylique, oléique et béhénylique, l'hexyldécanol, le 2-octyldodécanol, et leurs mélanges.

Les esters d'acide gras enC₈-C₄₀ et/ou d'alcool gras enC₈-C₄₀ peuvent être en particulier des mono-, di- ou polyester d'acide gras en C₈-C₄₀ et/ou d'alcool gras enC₈-C₄₀. Parmi ceux-ci, on préfère utiliser les monoesters d'alcool gras en C₈-C₄₀ tels que définis ci-dessus et/ou d'acide gras enC₈-C₄₀ tels que définis ci-dessous.

Les acides gras enC₈-C₄₀ peuvent être choisis parmi les acides de formule RCOOH, où R est un radical saturé ou insaturé, linéaire ou ramifié, comportant de 7 à 39 atomes de carbone. De préférence, R est un groupement alkyle en C₇-C₂₉ ou alcényle en C₇-C₂₉, mieux un groupement alkyle en C₁₂-C₂₄ ou alcényle en C₁₂-C₂₄. R peut être substitué par un ou plusieurs groupements hydroxy et/ou un ou plusieurs groupe carboxyle. L'acide gras peut être en particulier choisi parmi l'acide laurique, l'acide oléique, l'acide palmitique, l'acide linoléique, l'acide myristique et l'acide stéarique.

Les cires sont des substances naturelles (animales ou végétales) ou synthétiques solides à température ambiante (20°-25°C). Elles sont insolubles dans l'eau, solubles dans les huiles et sont capables de former un film hydrofuge.

Sur la définition des cires, on peut citer par exemple P.D. Dorgan, Drug and Cosmetic Industry, Decembre 1983, pp. 30-33.

La cire ou les cires éventuellement présentes dans la composition selon l'invention peuvent être choisies notamment, parmi la cire de Carnauba, la cire de Candelilla, la cire d'Alfa, la cire de paraffine, l'ozokérite, les cires végétales telles que la cire d'olivier, la cire de riz, la cire de jojoba hydrogénée ou les cires absolues de fleurs telles que la cire essentielle de fleur de cassis, les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines.

A titre d'huiles utilisables dans la composition de l'invention différentes des alcanes linéaires volatils, on peut citer par exemple :
- les huiles hydrocarbonées ramifiées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple l'huile de jojoba, l'huile d'avocat, l'huile de colza, l'huile d'olive, l'huile de tournesol, l'huile de maïs, l'huile de soja, l'huile de courge, l'huile de pépins de raisin, l'huile de sésame, l'huile de noisette, l'huile d'abricot, l'huile de macadamia, l'huile d'arara, l'huile de ricin, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;

- les hydrocarbures ramifiés, d'origine minérale ou synthétique, tels que les huiles de paraffine, volatiles ou non, et leurs dérivés, la vaseline, le polyisobutène hydrogéné tel que Parléam® ; les isoparaffines comme l'isohexadécane et l'isodécane.
- les huiles fluorées partiellement hydrocarbonées; comme huiles fluorées, on peut citer aussi le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

De préférence, le ou les corps gras ne comprennent pas de motif oxyalkyléné, ni de motif glycérolé.

Parmi tous les corps gras utilisables selon l'invention, on préfère choisir le ou les corps gras parmi les alcools gras cités ci-dessus et les cires telles que définies ci-avant.

Le ou les corps gras non siliconés différents des alcanes linéaires volatils représentent de 0,5 à 25%, de préférence de 1 à 10% en poids, du poids total de la composition.

La composition selon l'invention peut également comprendre un ou plusieurs polymères cationiques.

Par « polymère cationique », on entend au sens de la présente invention, tout polymère comprenant des groupements cationiques et/ou des groupements ionisables en groupements cationiques.

Le ou les polymères cationiques utilisables dans la composition cosmétique selon l'invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :
(1) les homopolymères ou copolymères d'esters ou d'amides acryliques ou méthacryliques, à fonctions aminées, comportant au moins un des motifs de formules suivantes : 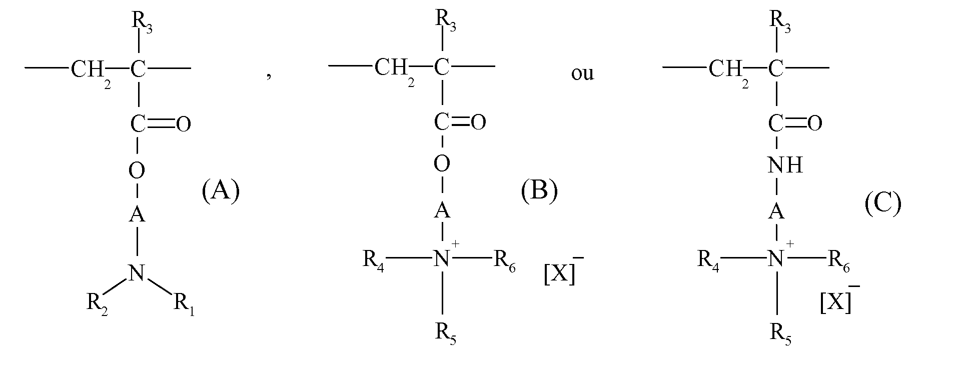 dans lesquelles:
   R₁ et R₂, identiques ou différents, représentent chacun un atome d'hydrogène ou un groupe alkyle ayant de 1 à 6 atomes de carbone ;
   R₃ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle ;
   X⁻ désigne un anion méthosulfate ou un halogénure tel que chlorure ou bromure.
Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C₁-₄), des groupes dérivés des acides acryliques ou méthacryliques ou de leurs esters, de vinyllactames tels que la vinylpyrrolidone ou le vinylcaprolactame, d'esters vinyliques.
Ainsi, parmi ces copolymères de la famille (1), on peut citer :
- les copolymères d'acrylamide et de méthacrylate de diméthylaminoéthyle quaternisés au sulfate de diméthyle ou avec un halogénure de diméthyle, tels que celui vendu sous la dénomination HERCOFLOC^{®} par la société HERCULES,
- les copolymères d'acrylamide et de chlorure de méthacryloyloxyéthyltriméthylammonium décrits, par exemple, dans la demande de brevet EP-A-080976 et vendus sous la dénomination BINA QUAT P 100 par la société CIBA GEIGY,
- les copolymères d'acrylamide et de méthosulfate de méthacryloyloxyéthyltriméthylammonium, tels que celui vendu sous la dénomination RETEN par la société HERCULES,
- les copolymères vinylpyrrolidone/acrylate ou méthacrylate de dialkylaminoalkyle quaternisés ou non, tels que les produits vendus sous la dénomination "GAFQUAT^{®}" par la société ISP comme, par exemple, "GAFQUAT^{®} 734" ou "GAFQUAT^{®} 755", ou bien les produits dénommés "COPOLYMER^{®} 845, 958 et 937". Ces polymères sont décrits en détail dans les brevets français Nos 2 077 143 et 2 393 573,
- les terpolymères méthacrylate de diméthylaminoéthyle/vinylcaprolactame/vinylpyrrolidone tels que le produit commercialisé sous la dénomination GAFFIX^{®} VC 713 par la société ISP, et
- les copolymères vinylpyrrolidone/méthacrylamide de diméthylaminopropyle quaternisé tels que notamment le produit commercialisé sous la dénomination "GAFQUAT^{®} HS 100" par la société ISP ;
- les polymères réticulés de sels de méthacryloyloxyalkyl(C1-C4) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société CIBA.
(2) les polysaccharides cationiques, et en particulier ceux choisis parmi :
   a) les dérivés d'éthers de cellulose comportant des groupements ammonium quaternaire décrits dans le brevet FR 1492597. Ces polymères sont également définis dans le dictionnaire CTFA comme des ammoniums quaternaires d'hydroxyéthyl cellulose ayant réagi avec un époxyde substitué par un groupement triméthylammonium ;
   b) les dérivés de cellulose greffés avec un monomère hydrosoluble comportant un ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyloxyéthyl-triméthylammonium, de méthacrylamidopropyl triméthylammonium, de diméthyl-diallylammonium.
      Les produits commercialisés répondant à cette définition sont plus particulièrement les produits vendus sous la dénomination "CELQUAT L 200" et "CELQUAT H 100" par la Société National Starch.
   c) les polygalactomananes cationiques tels que ceux décrits dans les brevets américains 3 589 578 et 4 031 307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL ;
(3) les copolymères quaternaires de vinylpyrrolidone et de vinylimidazole ;
(4) les chitosanes ou leurs sels ; les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90,5 % en poids vendu sous la dénomination KYTAN BRUT STANDARD par la société ABER TECHNOLOGIES, le pyrrolidone-carboxylate de chitosane commercialisé sous la dénomination KYTAMER^{®} PC par la société AMERCHOL.

Parmi tous les polymères cationiques pouvant être présents dans la composition selon l'invention, on préfère choisir le ou les polymères cationiques parmi les polymères réticulés de sels de méthacryloyloxyalkyl(C1-C4) trialkyl(C1-C4)ammonium, et les polysaccharides cationiques.

Lorsque la composition comprend au moins un polymère cationique, celui-ci ou ceux-ci sont présents dans une concentration allant de préférence de 0,01 à 10% en poids du poids total de la composition.

La composition selon l'invention peut comprendre en outre un ou plusieurs tensioactifs choisis parmi des tensioactifs anioniques, cationiques, non ioniques ou amphotères.

De préférence, le ou les tensioactifs sont cationiques.

On entend par « tensioactif cationique », un tensioactif chargé positivement lorsqu'il est contenu dans la composition selon l'invention. Ce tensioactif peut porter une ou plusieurs charges permanentes positives ou contenir une ou plusieurs fonctions cationisables au sein de la composition selon l'invention. A titre d'exemple de tensioactifs cationiques utilisables dans la composition cosmétique, on peut notamment citer les amines grasses primaires, secondaires ou tertiaires, éventuellement polyoxyalkylénées, ou leurs sels, les sels d'ammonium quaternaire, et leurs mélanges.

Les amines grasses comprennent en général au moins une chaîne hydrocarbonée en C₈-C₃₀. Parmi les amines grasses utilisables selon l'invention, on peut citer par exemple la stéaryl amidopropyl diméthylamine.

Les amines grasses comprennent en général au moins une chaîne hydrocarbonée en C₈-C₃₀. Parmi les amines grasses utilisables selon l'invention, on peut citer par exemple la stéaryl amidopropyl diméthylamine et la distéarylamine.

A titre de sels d'ammonium quaternaires, on peut notamment citer, par exemple :
- ceux qui présentent la formule générale (I) suivante : 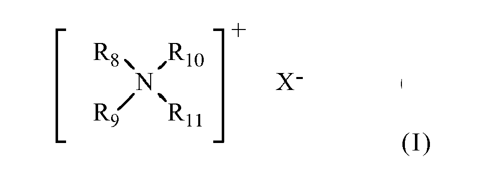 dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle, au moins un des radicaux R₈ à R₁₁ comportant de 8 à 30 atomes de carbones, de préférence de 12 à 24 atomes de carbone. Les radicaux aliphatiques peuvent comporter des hétéroatomes tels que notamment l'oxygène, l'azote, le soufre et les halogènes.
   Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline, comme par exemple ceux de formule (II) suivante : 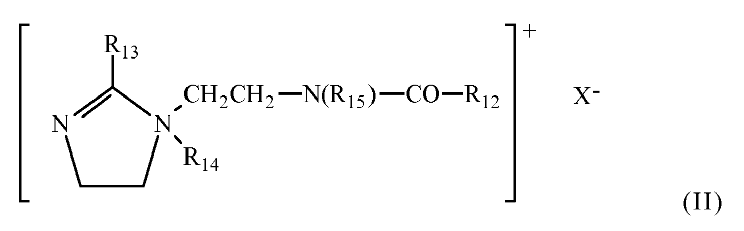 dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT^{®} W 75 par la société REWO ;
- les sels de diammonium quaternaire de formule (III) : 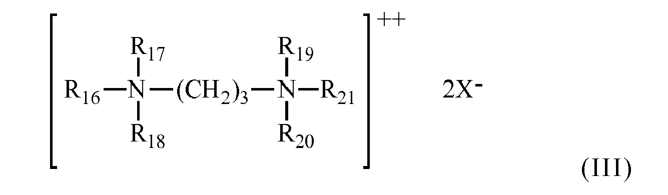 dans laquelle R₁₆ désigne un radical aliphatique comportant environ de 16 à 30 atomes de carbone, R₁₇, R₁₈, R₁₉, R₂₀ et R₂₁, identiques ou différents sont choisis parmi l'hydrogène ou un radical alkyle comportant de 1 à 4 atomes de carbone, et X⁻ est un anion choisi dans le groupe des halogénures, acétates, phosphates, nitrates et méthylsulfates. De tels sels de diammonium quaternaire comprennent notamment le dichlorure de propane suif diammonium ;
- les sels d'ammonium quaternaire contenant au moins une fonction ester, tels que ceux de formule (IV) suivante : 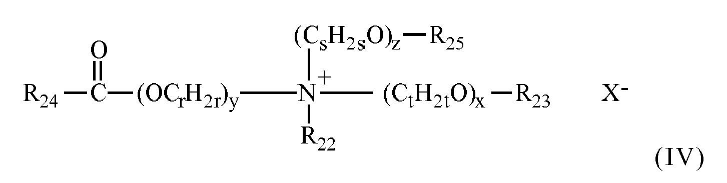 dans laquelle :
   R₂₂ est choisi parmi les radicaux alkyles en C₁-C₆ et les radicaux hydroxyalkyles ou dihydroxyalkyles en C₁-C₆ ;
   R₂₃ est choisi parmi :
- le radical 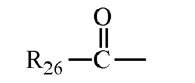
- les radicaux R₂₇ qui sont des radicaux hydrocarbonés en C₁-C₂₂, linéaires ou ramifiés, saturés ou insaturés,
- l'atome d'hydrogène,
   R₂₅ est choisi parmi :
- le radical 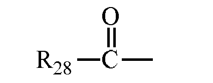
- les radicaux R₂₉ qui sont des radicaux hydrocarbonés en C₁-C₆, linéaires ou ramifiés, saturés ou insaturés,
- l'atome d'hydrogène,
   R₂₄, R₂₆ et R₂₈, identiques ou différents, sont choisis parmi les radicaux hydrocarbonés en C₇-C₂₁, linéaires ou ramifiés, saturés ou insaturés ;
   r, s et t, identiques ou différents, sont des entiers valant de 2 à 6 ;
   y est un entier valant de 1 à 10 ;
   x et z, identiques ou différents, sont des entiers valant de 0 à 10 ;
   X⁻ est un anion simple ou complexe, organique ou inorganique ;
sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion X⁻ est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IV) dans laquelle :
- R₂₂ désigne un radical méthyle ou éthyle,
- x et y sont égaux à 1;
- z est égal à 0 ou 1 ;
- r, s et t sont égaux à 2;
- R₂₃ est choisi parmi :
   - le radical 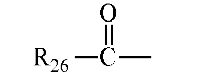
   - les radicaux méthyle, éthyle ou hydrocarbonés en C₁₄-C₂₂,
   - l'atome d'hydrogène ;
   - R₂₅ est choisi parmi :
   - le radical 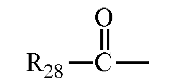
   - l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (IV) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxy éthyl-méthylammonium, de monoacyloxyéthyl-hydroxyéthyl-diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthane sulfonate de méthyle, le paratoluènesulfonate de méthyle, la chlorhydrine du glycol ou du glycérol.

De tels composés sont par exemple commercialisés sous les dénominations DEHYQUART^{®} par la société HENKEL, STEPANQUAT^{®} par la société STEPAN, NOXAMIUM^{®} par la société CECA, REWOQUAT^{®} WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthyl sulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (I), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le méthosulfate de distéaroyléthyl hydroxyéthyl méthylammonium, le méthosulfate de dipalmitoyléthylhydroxyéthylammonium ou le méthosulfate de dicétylaroyléthylhydroxyéthylammonium, ou encore, enfin, le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK.

Parmi tous les tensioactifs cationiques pouvant être présents dans la composition selon l'invention, on préfère choisir le ou les tensioactifs cationiques parmi les sels de (chlorure ou méthosulfate) de cétyltriméthylammonium (INCI cetrimonium-), de béhényltriméthylammonium (INCI behentrimonium-), de dipalmitoyléthylhydroxyéthylammonium, de distéaroyléthylhydroxyéthyl méthylammonium, de méthyl alkyl(C₉-C₁₉) alkyl(C₁₀-C₂₀)amidoéthylimidazolium, la stéaramidopropyldiméthylamine, le sel de stéaramidopropyldiméthylammonium, et leurs mélanges.

Lorsque la composition comprend au moins un tensioactif cationique, celui-ci ou ceux-ci sont présents dans une concentration allant de préférence de 0,1 à 10% en poids du poids total de la composition.

La composition selon l'invention comprend un milieu aqueux.

Le milieu aqueux est constitué d'eau ou d'un mélange d'eau et d'au moins un solvant cosmétiquement acceptable de préférence choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges. Lorsque que le milieu comprend au moins un solvant, il comprend majoritairement de l'eau, c'est-à-dire au moins 50% en poids d'eau par rapport au poids total du milieu aqueux.

La composition selon l'invention peut comprendre en outre un ou plusieurs additifs classiques bien connus dans la technique, différents des composés définis précédemment. A titre d'exemples d'additifs utilisable selon l'invention, on peut citer des polymères associatifs ou non, ioniques ou non-ioniques, des filtres, des silanes, des silicones de viscosité inférieure à 20 000 mm²/s, des polyols, des protéines, des vitamines, des agents réducteurs, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des agents nacrants, des propulseurs, et des épaississants minéraux ou organiques ; ces additifs étant différents des composés définis plus haut.

L'homme de métier veillera à choisir le ou les éventuels additifs et leur quantité de manière à ce qu'ils ne nuisent pas aux propriétés des compositions de la présente invention.

Le ou les additifs sont généralement présents dans la composition selon l'invention en une quantité allant de 0 à 20 % en poids par rapport au poids total de la composition.

Les compositions selon l'invention peuvent se présenter sous la forme de composition de soin rincé ou non-rincé, ces dernières se présentant sous la forme d'une lotion plus ou moins épaissie, d'une crème, d'un gel ou d'une émulsion.

Un autre objet de l'invention est l'utilisation de la composition cosmétique telle que décrite ci-dessus pour le traitement cosmétique des matières kératiniques, de préférence des fibres kératiniques comme les cheveux, et notamment comme produit capillaire rincé.

L'invention est également relative à un procédé de traitement cosmétique des matières kératiniques, de préférence des fibres kératiniques comme les cheveux, qui comprend l'application d'une quantité efficace d'une composition cosmétique telle que décrite ci-dessus, sur lesdites matières, et éventuellement le rinçage de ladite composition après un éventuel temps de pause.

Lorsque l'on applique la composition selon l'invention sous forme d'une lotion ou d'une crème, on la laisse éventuellement pauser sur les cheveux pendant environ 0,5 à 5 minutes, puis on rince éventuellement à l'eau.

Les exemples suivants sont donnés à titre illustratif de la présente invention.

Dans les exemples suivants, toutes les quantités sont indiquées en pour cent en poids en produit en l'état par rapport au poids total de la composition, sauf indication contraire.

### EXEMPLES

On a préparé les compositions de soin rincé A, B et C suivantes à partir des ingrédients indiqués dans le tableau ci-dessous.

| Compositions | A | B | c |
|---|---|---|---|
| Mélange de n-undécane et de n-tridécane selon l'exemple 2 du WO 2008/155059 | 5 | 3 | - |
| Mélange de dodécane et de tétradécane pur (85/15), vendu sous la dénomination commerciale VEGELIGHT par la Biosynthis | - | - | 5 |
| Copolymère polydiméthylsiloxane à groupement alpha-oméga vinyle/ alpha-oméga hydrogénopolysiloxane en émulsion cationique, vendu sous la dénomination | 2 | 2 | 2 |
| commerciale Dow Corning 2-1997 par la société Dow Corning (viscosité d'environ 20.10⁶ mm²/s) 67% en poids de matière active | | | |
| Alcool myristylique, vendu sous la dénomination commerciale Nacol 14-98 par la société Sasol | 3 | 3 | 3 |
| Alcool cétylique, vendu sous la dénomination commerciale Nacol 16-98 par la société Sasol | 1 | 1 | 1 |
| Homopolymère de chlorure de méthacrylate d'éthyl triméthyl ammonium réticulé, en émulsion inverse à 50% en poids dans une huile minérale, vendu sous la dénomination commerciale SALCARE® SC 95 par la société Ciba | 0,5 | 0,5 | 0,5 |
| Stéaryl amidopropyl diméthylamine, vendu sous la dénomination commerciale Mackine 301 par la société Rhodia | 2,5 | 2,5 | 2,5 |
| Méthosulfate de distéaroyléthyl hydroxyéthyl méthyl ammonium à 75% en poids dans de l'alcool stéarylique, vendu sous la dénomination commerciale Dehyquart F75 par la société Cognis | 2,5 | 2,5 | 2,5 |
| Lauryl PEG/PPG-18/18 methicone à 72% en poids de matière active, vendu sous la dénomination commerciale Dow Corning 5200 formulation aid par la société Dow Corning (viscosité 2750 MM²/S) | 1 | 1 | 1 |
| Acide citrique, 1 H₂O | 0,85 | 0,85 | 0,85 |
| Conservateur, Parfum | qs | qs | qs |
| Eau désionisée | Qs 100 | Qs 100 | Qs 100 |

Les compositions A, B et C selon l'invention présentent de bonnes propriétés de lissage de la fibre capillaire lors du rinçage et sur cheveux séchés.

## Revendications

1. Composition cosmétique comprenant dans un milieu aqueux:
- de 0,1 à 20% en poids d'un ou plusieurs alcanes linéaires volatils,
- de 0,5 à 25% en poids d'une ou plusieurs silicones de viscosité supérieure ou égale à 20 000 mm²/s à 25°C,
- un ou plusieurs corps gras non siliconés différents des alcanes linéaires volatils,
le rapport pondéral de la quantité dudit ou desdits corps gras non siliconés sur la quantité de ladite ou desdites silicones étant inférieur ou égal à 5.

2. Composition cosmétique selon la revendication précédente, **caractérisée en ce que** le ou les alcanes linéaires volatils sont les alcanes linéaires comportant de 7 à 15 atomes de carbone.

3. Composition cosmétique selon la revendication 1 ou 2, **caractérisée en ce que** le ou les alcanes linéaires volatils sont choisis parmi le n-nonane, le n-undécane, le n-dodécane, le n-tridécane, le n-tétradécane, et leurs mélanges.

4. Composition cosmétique selon l'une quelconque des revendications précédentes, **caractérisée en ce que** le ou les alcanes linéaires volatils sont présents en une teneur allant de 1 à 20% en poids, de préférence de 1 à 15%, et mieux de 3 à 15% en poids par rapport au poids total de la composition

5. Composition cosmétique selon l'une quelconque des revendications précédentes, **caractérisée en ce que** la ou les silicones sont choisies parmi :
(i) les polyalkylsiloxanes, tels que les polydiméthylsiloxanes linéaires à groupements terminaux triméthylsilyle et les polydiméthylsiloxanes à groupements terminaux hydroxydiméthysilyle.
(ii) les polyarylsiloxanes ;
(iii) les polyalkylarylsiloxanes, tels que les polyméthylphénylsiloxanes, les polydiméthylméthylphénylsiloxanes, les polydiméthyldiphénylsiloxanes linéaires et ramifiés,
(iv) les gommes de silicone des silicones (i), (ii) et (iii),
(v) les résines de silicones,
(vi) et leurs mélanges.

6. Composition cosmétique selon la revendication précédente, **caractérisée en ce que** la ou les silicones sont choisies parmi les gommes de silicone telles que définies dans la revendication précédente.

7. Composition cosmétique selon la revendication 5 ou 6, **caractérisée en ce que** les gommes de silicones sont choisies parmi les gommes possédant l'une des structures suivantes:
- polydiméthylsiloxane,
- poly[(diméthylsiloxane)/(méthylvinylsiloxane)],
- poly[(diméthylsiloxane)/(vinylhydrogénosiloxane)],
- poly[(dihydrogénodiméthylsiloxane)/(divinylsiloxane)],
- poly[(diméthylsiloxane)/(diphénylsiloxane)],
- poly[(diméthylsiloxane))(phénylméthylsiloxane)],
- poly[(diméthylsiloxane)/(diphénylsiloxane)/(méthylvinylsiloxane)];
et leurs mélanges.

8. Composition cosmétique selon l'une quelconque des revendications précédentes, **caractérisée en ce que** la ou les silicones sont présentes en une teneur allant de 0,1 à 10 % en poids, de préférence de 0,5 à 5% en poids, par rapport au poids total de la composition.

9. Composition cosmétique selon l'une quelconque des revendications précédentes, **caractérisée en ce que** le ou les corps gras non siliconés différents des alcanes linéaires volatils sont choisis parmi les alcools gras en C₈-C₄₀, les esters d'acide gras en C₇-C₃₉ et/ou d'alcool gras en C₈-C₄₀, les cires, les huiles végétales, animales, minérales et synthétiques.

10. Composition cosmétique selon l'une quelconque des revendications précédentes, **caractérisée en ce que** le ou les corps gras non siliconés différents des alcanes linéaires volatils sont choisis parmi les alcools gras de formule R'OH, où R' désigne un radical saturé ou insaturé, linéaire ou ramifié, comportant de 8 à 40 atomes de carbone, de préférence de 8 à 30 atomes de carbone.

11. Composition cosmétique selon l'une quelconque des revendications précédentes, **caractérisée en ce que** le ou les corps gras différents des alcanes linéaires volatils sont présents en une teneur allant de 0,5 à 25% en poids, de préférence de 1 à 10% en poids, par rapport au poids total de la composition.

12. Composition cosmétique selon l'une quelconque des revendications précédentes, **caractérisée en ce que** le rapport pondéral de la quantité de corps gras non siliconé(s) différents des alcanes linéaires volatils sur la quantité de silicone(s) de viscosité supérieure ou égale à 20 000 mm²/s va de 0,01 à 5, mieux encore de 0,1 à 5.

13. Composition cosmétique selon l'une quelconque des revendications précédentes, **caractérisée en ce qu'**elle comprend en outre au moins un polymère cationique et/ou au moins un tensioactif cationique.

14. Utilisation de la composition cosmétique selon l'une quelconque des revendications précédentes, pour le traitement des matières kératiniques, de préférence des cheveux.

15. Procédé de traitement cosmétique des matières kératiniques, de préférence des fibres kératiniques, et mieux encore des cheveux, comprenant l'application de la composition cosmétique selon l'une quelconque des revendications 1 à 13.
